# EUROPEAN PATENT SPECIFICATION

(11) **EP 2 760 422 B1**
(45) Date of publication and mention of the grant of the patent: **06.09.2017**
(21) Application number: 12769391.9
(22) Date of filing: 28.09.2012
(51) Int. Cl.: A61K 8/34, A61K 8/81, A61Q 5/10

(54) **FOAM DYE COMPOSITION COMPRISING AT LEAST ONE LIQUID FATTY ALCOHOL AND A PARTICULAR CATIONIC POLYMER**
SCHAUMFÄRBEZUSAMMENSETZUNG MIT MINDESTENS EINEM FLÜSSIGEN FETTALKOHOL UND EINEM BESTIMMTEN KATIONISCHEN POLYMER
COMPOSITION DE MOUSSE COLORANTE COMPRENANT AU MOINS UN ALCOOL GRAS LIQUIDE ET UN POLYMÈRE CATIONIQUE PARTICULIER

(30) Priority: 30.09.2011 FR 1158841; 18.10.2011 US 201161548335 P
(43) Date of publication of application: 06.08.2014
(73) Proprietor: L'Oréal, 75008 Paris (FR)
(72) Inventor: GOGET, Caroline, 07901 Summit, NJ (US); ALLARD, Delphine, F-75009 Paris (FR); SABOURIN, Richard, F-75005 Paris (FR)
(74) Representative: Fevrier, Murielle Françoise E.
(86) International application number: PCT/EP2012/069215
(87) International publication number: WO 2013/045630

(56) References cited:
- EP-A1- 2 204 157
- EP-A1- 2 283 803
- FR-A1- 2 833 832
- US-A1- 2004 098 815
- US-A1- 2010 236 570
- US-B2- 7 799 746
- WU W ET AL: "QUANTITATIVE METHODS FOR EVALUATING OPTICAL AND FRICTIONAL PROPERTIES OF CATIONIC POLYMERS", JOURNAL OF COSMETIC SCIENCE, SOCIETY OF COSMETIC CHEMISTS, NEW YORK, NY, US, vol. 52, no. 1, 1 January 2001 (2001-01-01), pages 51-65, XP001096307, ISSN: 1525-7886

## Description

The present invention relates to a dye composition in foam form.

Among the methods for dyeing human keratin fibres, such as the hair, mention may be made of oxidation dyeing or permanent dyeing. More particularly, this form of dyeing uses one or more oxidation dyes, usually one or more oxidation bases optionally combined with one or more couplers.

In general, oxidation bases are chosen from ortho- or para-phenylenediamines, ortho- or para-aminophenols and heterocyclic compounds. These oxidation bases are colourless or weakly coloured compounds which, when combined with oxidizing products, can give access to coloured entities.

The shades obtained with these oxidation bases are often varied by combining them with one or more couplers, these couplers being chosen especially from aromatic meta-diamines, meta-aminophenols, meta-diphenols and certain heterocyclic compounds, such as indole compounds.

The variety of molecules used as oxidation bases and couplers allows a wide range of colors to be obtained.

Permanent dyeing processes thus consist in using, with the dye composition, an aqueous composition comprising at least one oxidizing agent, such as hydrogen peroxide, under alkaline pH conditions in the vast majority of cases. The alkaline agent conventionally used is aqueous ammonia or other alkaline agents, such as alkanolamines.

Dye compositions may take various forms such as lotions, gels, emulsions, creams or foams. Dyeing foams are pleasant to use, however they often exhibit a poor staying power over time. For example, it is possible to observe a rapid disappearance of the foam after application or a non-uniform application along the fibres. EP 2 283 803 discloses a two-part foam hair dye having a first agent containing an oxidation dye, an alkali agent, a water-soluble cationic polymer and a solid higher alcohol. There is a real, constant need to develop oxidation dye compositions in foam form that are easy to prepare and to apply, and which remain sufficiently stable over time while retaining efficient dyeing properties.

This aim and others are achieved by the present invention, one subject of which is thus a composition for dyeing keratin fibres such as the hair, in foam form, comprising at least one alkaline agent selected from ammonia and monoethanolamine, and mixtures thereof, at least one oxidizing agent, at least one oxidation dye precursor and at least one liquid fatty alcohol containing from 8 to 30 carbon atoms and at least one cationic polymer of formula (I) or (II) with a cationic charge density of greater than 4 meq./g.

The invention also relates to a process for dyeing human keratin fibres using this composition.

A subject of the invention is similarly a multi-compartment device comprising, in a first compartment, a first composition containing at least one oxidation dye precursor, at least one alkaline agent, at least one liquid fatty alcohol and at least one cationic polymer with a cationic charge density of greater than 4 meq./g; in a second compartment, a second composition containing one or more oxidizing agents, one of the two compartments being equipped with a component for delivering the composition of the invention in foam form after mixing with the other composition, or the component for delivering the composition in foam form is included in a third compartment, the composition being as above defined.

Preferably, the fatty alcohol and the cationic polymer as above defined are present in the composition containing the oxidation dye precursor(s).

A subject of the invention is also a device for dyeing keratin fibres, comprising the composition of the invention in liquid form and a foam dispenser for delivering the composition in the form of a foam.

The composition of the invention is in the form of a foam that is particularly pleasant to apply. It has a light, airy texture, which makes it particularly pleasant to use. The qualities of the foam are sufficiently long-lasting to enable uniform application of the dye product, without running. The composition of the invention makes it possible to obtain improved dyeing properties, such as strength of the colour, resistance to external agents (shampooing, perspiration, light) and selectivity, which are particularly efficient.

Other features and advantages of the invention will become more clearly apparent on reading the description and the examples that follow.

In the text hereinbelow, unless otherwise indicated, the limits of a range of values are included in that range. The expression "at least one" is equivalent to the expression "one or more".

The composition of the invention comprises at least one liquid fatty alcohol. The term "liquid fatty alcohol" means a non-glycerolated and non-oxyalkylenated fatty alcohol which is liquid at standard temperature (25°C) and at atmospheric pressure (760 mmHg; i.e. 1.013×10⁵ Pa).

The liquid fatty alcohols of the invention may be saturated or unsaturated.

The saturated liquid fatty alcohols are preferably branched. They may optionally comprise in their structures at least one aromatic or non-aromatic ring. They are preferably acyclic.

More particularly, the liquid saturated fatty alcohols of the invention are chosen from octyldodecanol, isostearyl alcohol and 2-hexyldecanol.

Octyldodecanol is most particularly preferred.

The liquid unsaturated fatty alcohols have in their structure at least one double or triple bond. Preferably, the fatty alcohols of the invention bear in their structure one or more double bonds. When several double bonds are present, there are preferably 2 or 3 of them, and they may be conjugated or non-conjugated.

These unsaturated fatty alcohols may be linear or branched.

They can optionally comprise, in their structures, at least one aromatic or nonaromatic ring. They are preferably acyclic.

More particularly, the liquid unsaturated fatty alcohols of the invention are selected from oleic (or oleyl) alcohol, linoleic (or linoleyl) alcohol, linolenic (or linolenyl) alcohol and undecylenyl alcohol.

In the composition of the invention, oleyl alcohol is most particularly preferred as liquid unsaturated fatty alcohol.

In the composition of the invention, the liquid fatty alcohol(s) are generally present in an amount ranging from 0.1 % to 20%, preferably from 0.2% to 10% and better still from 0.3% to 5% by weight, relative to the total weight of the composition.

The composition of the invention comprises at least one cationic polymer with a cationic charge density of greater than than 4 meq./g.

This charge density is especially determined by the Kjeldahl method. It may also be calculated from the chemical nature of the polymer.

These polymers preferably have a number-average molecular mass of between 1000 and 100 000.

Polymers of this type are described in particular in FR 2 320 330, FR 2 270 846, FR 2 316 271, FR 2 336 434, FR 2 413 907, US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 and US 4 027 020.

More particularly, the cationic polymer(s) are chosen from:
a) cyclopolymers of alkyldiallylamine or of dialkyldiallylammonium, such as homopolymers or copolymers containing, as main constituent of the chain, units corresponding to formula (I) or (II): 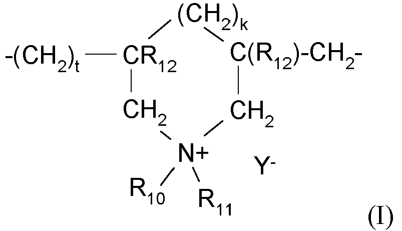 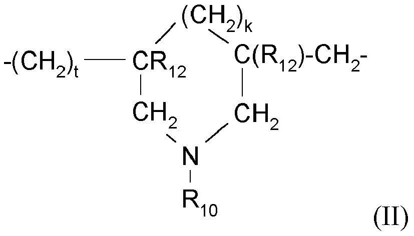 in which formulae k and t are equal to 0 or 1, the sum k + t being equal to 1; R₁₂ denotes a hydrogen atom or a methyl radical; R₁₀ and R₁₁, independently of each other, denote an alkyl group containing from 1 to 6 carbon atoms, a hydroxyalkyl group in which the alkyl group preferably contains 1 to 5 carbon atoms, a lower (C₁-C₄) amidoalkyl group, or R₁₀ and R₁₁ can denote, together with the nitrogen atom to which they are attached, heterocyclic groups such as piperidyl or morpholinyl; Y⁻ is an anion such as bromide, chloride, acetate, borate, citrate, tartrate, bisulfate, bisulfite, sulfate or phosphate.

These polymers are in particular described in French patent 2 080 759 and in its Certificate of Addition 2 190 406.

R₁₀ and R₁₁, independently of each other, preferably denote an alkyl group containing from 1 to 4 carbon atoms.

Among the polymers defined above, mention may be made more particularly of the dimethyldiallylammonium halide homopolymer sold under the name Merquat 100 by the company Nalco (and its homologues of low weight-average molecular mass) and the copolymers of diallyldimethylammonium halide and of acrylamide, sold under the name Merquat 550.

The amount of cationic polymer(s) that is useful in the composition of the invention generally ranges from 0.01% to 10%, preferably from 0.1 % to 5% and better still from 0.2% to 2% by weight relative to the total weight of the composition.

The foam composition according to the invention comprises at least one alkaline agent monoethanolamine, and aqueous ammonia and mixtures thereof. In this variant, the alkanolamine(s) are present in a predominant amount relative to the aqueous ammonia, the content of the latter being expressed as ammonia.

Advantageously, the composition according to the invention has a content of alkaline agent(s) ranging from 0.01% to 30% by weight, preferably from 0.1 % to 20% by weight and better still from 1% to 10% by weight relative to the weight of the said composition.

The composition according to the invention also comprises at least one oxidizing agent.

The oxidizing agents are chosen, for example, from hydrogen peroxide, urea peroxide, alkali metal bromates or ferricyanides, peroxygenated salts, for instance persulfates, perborates, peracids and precursors thereof and percarbonates of alkali metals or of alkaline-earth metals. Advantageously, the oxidizing agent is hydrogen peroxide.

The content of oxidizing agent(s) more particularly represents from 0.1 % to 20% by weight and preferably from 0.5% to 10% by weight relative to the weight of the composition.

As indicated previously, the composition according to the invention comprises one or more oxidation dye precursors.

Oxidation bases and couplers may be used as oxidation dye precursors.

By way of example, the oxidation bases are chosen from para-phenylenediamines, bis(phenyl)alkylenediamines, para-aminophenols, ortho-aminophenols and heterocyclic bases, and the addition salts thereof.

Among the para-phenylenediamines that may be mentioned, for example, are *para*-phenylenediamine, *para*-toluenediamine, 2-chloro-para-phenylenediamine, 2,3-dimethyl-para-phenylenediamine, 2,6-dimethyl-*para*-phenylenediamine, 2,6-diethyl-*para*-phenylenediamine, 2,5-dimethyl-*para*-phenylenediamine, N,N-dimethyl-*para-*phenylenediamine, N,N-diethyl-*para*-phenylenediamine, N,N-dipropyl-*para-*phenylenediamine, 4-amino-N,N-diethyl-3-methylaniline, N,N-bis(β-hydroxyethyl)-*para*-phenylenediamine, 4-N,N-bis(β-hydroxyethyl)amino-2-methylaniline, 4-N,N-bis(β-hydroxyethyl)amino-2-chloroaniline, 2-β-hydroxyethyl-*para*-phenylenediamine, 2-fluoro-*para*-phenylenediamine, 2-isopropyl-*para*-phenylenediamine, N-(β-hydroxypropyl)-*para*-phenylenediamine, 2-hydroxymethyl-*para*-phenylenediamine, N,N-dimethyl-3-methyl-*para*-phenylenediamine, N-ethyl-N-(β-hydroxyethyl)-*para-*phenylenediamine, N-(β,γ-dihydroxypropyl)-*para*-phenylenediamine, N-(4'-aminophenyl)-*para*-phenylenediamine, N-phenyl-*para*-phenylenediamine, 2-β-hydroxyethyloxy-*para*-phenylenediamine, 2-β-acetylaminoethyloxy-*para-*phenylenediamine, N-(β-methoxyethyl)-*para*-phenylenediamine, 4-aminophenylpyrrolidine, 2-thienyl-*para*-phenylenediamine, 2-β-hydroxyethylamino-5-aminotoluene, 3-hydroxy-1-(4'-aminophenyl)pyrrolidine, and the addition salts thereof with an acid.

Among the para-phenylenediamines mentioned above, para-phenylenediamine, para-toluenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and the addition salts thereof with an acid, are particularly preferred.

Among the bis(phenyl)alkylenediamines that may be mentioned, for example, are N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N, N'-bis(4-methylaminophenyl)tetramethylenediamine, N,N'-bis(ethyl)-N,N'-bis(4'-amino-3'-methylphenyl)ethylenediamine and 1,8-bis(2,5-diaminophenoxy)-3,6-dioxaoctane, and the addition salts thereof.

Among the para-aminophenols that may be mentioned, for example, are para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

Among the ortho-aminophenols that may be mentioned, for example, are 2-aminophenol, 2-amino-5-methylphenol, 2-amino-6-methylphenol and 5-acetamido-2-aminophenol, and the addition salts thereof.

Among the heterocyclic bases, mention may be made, by way of example, of pyridine derivatives, pyrimidine derivatives and pyrazole derivatives.

Among the pyridine derivatives that may be mentioned are the compounds described, for example, in patents GB 1 026 978 and GB 1 153 196, for instance 2,5-diaminopyridine, 2-(4-methoxyphenyl)amino-3-aminopyridine and 3,4-diaminopyridine, and the addition salts thereof.

Other pyridine oxidation bases of use in the present invention are the 3-aminopyrazolo[1,5-a]pyridine oxidation bases or the addition salts thereof described, for example, in Patent Application FR 2 801 308. Examples that may be mentioned include pyrazolo[1,5-a]pyrid-3-ylamine, 2-acetylaminopyrazolo[1,5-a]pyrid-3-ylamine, 2-(morpholin-4-yl)pyrazolo[1,5-a]pyrid-3-ylamine, 3-aminopyrazolo[1,5-a]pyridine-2-carboxylic acid, 2-methoxypyrazolo[1,5-a]pyrid-3-ylamine, (3-aminopyrazolo[1,5-a]pyrid-7-yl)methanol, 2-(3-aminopyrazolo[1,5-a]pyrid-5-yl)ethanol, 2-(3-aminopyrazolo[1,5-a]pyrid-7-yl)ethanol, (3-aminopyrazolo[1,5-a]pyrid-2-yl)methanol, 3,6-diaminopyrazolo[1,5-a]pyridine, 3,4-diaminopyrazolo[1,5-a]pyridine, pyrazolo[1,5-a]pyridine-3,7-diamine, 7-(morpholin-4-yl)pyrazolo[1,5-a]pyrid-3-ylamine, pyrazolo[1,5-a]pyridine-3,5-diamine, 5-(morpholin-4-yl)pyrazolo[1,5-a]pyrid-3-ylamine, 2-[(3-aminopyrazolo[1,5-a]pyrid-5-yl)(2-hydroxyethyl)amino]ethanol, 2-[(3-aminopyrazolo[1,5-a]pyrid-7-yl)(2-hydroxyethyl)amino]ethanol, 3-aminopyrazolo[1,5-a]pyridin-5-ol, 3-aminopyrazolo[1,5-a]pyridin-4-ol, 3-aminopyrazolo[1,5-a]pyridin-6-ol and 3-aminopyrazolo[1,5-a]pyridin-7-ol, and addition salts thereof.

Among the pyrimidine derivatives that may be mentioned are the compounds described, for example, in patents DE 2359399, JP 88-169571, JP 05-63124 and EP 0 770 375 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine and addition salts thereof, and tautomeric forms thereof, when a tautomeric equilibrium exists.

Among the pyrazole derivatives that may be mentioned are the compounds described in the patents DE 3843892, DE 4133957 and patent applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-*tert*-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole, 3,5-diamino-4-(β-hydroxyethyl)amino-1-methylpyrazole, and the addition salts thereof. Use may also be made of 4,5-diamino-1-(β-methoxyethyl)pyrazole.

Use will preferably be made of a 4,5-diaminopyrazole and more preferably still of 4,5-diamino-1-(β-hydroxyethyl)pyrazole and/or a salt thereof.

Pyrazole derivatives that may also be mentioned include diamino-N,N-dihydropyrazolopyrazolones and especially those described in patent application FR-A-2 886 136, such as the following compounds and the addition salts thereof: 2,3-diamino-6,7-dihydro-1H,5H-pyrazolo[1,2-a]pyrazol-1-one, 2-amino-3-ethylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-dimethyl-1,2-dihydropyrazol-3-one, 4,5-diamino-1,2-diethyl-1,2-dihydropyrazol-3-one, 4,5-diamino-1,2-di(2-hydroxyethyl)-1,2-dihydropyrazol-3-one, 2-amino-3-(2-hydroxyethyl)amino-6,7-dihydro-1H,5H-pyrazolo[1,2-a]pyrazol-1-one, 2-amino-3-dimethylamino-6,7-dihydro-1H,5H-pyrazolo[1,2-a]pyrazol-1-one, 2,3-diamino-5,6,7,8-tetrahydro-1H,6H-pyridazino[1,2-a]pyrazol-1-one, 4-amino-1,2-diethyl-5-(pyrrolidin-1-yl)-1,2-dihydropyrazol-3-one, 4-amino-5-(3-dimethylaminopyrrolidin-1-yl)-1,2-diethyl-1,2-dihydropyrazol-3-one, 2,3-diamino-6-hydroxy-6,7-dihydro-1H,5H-pyrazolo[1,2-a]pyrazol-1-one. 2,3-Diamino-6,7-dihydro-1H,5H-pyrazolo[1,2-a]pyrazol-1-one and/or a salt thereof will preferably be used.

As heterocyclic bases, use will preferably be made of 4,5-diamino-1-(β-hydroxyethyl)pyrazole and/or 2,3-diamino-6,7-dihydro-1H,5H-pyrazolo[1,2-a]pyrazol-1-one and/or a salt thereof.

Among the couplers that may be used in the composition of the invention, mention may be made especially of meta-phenylenediamines, meta-aminophenols, meta-diphenols, naphthalene-based couplers and heterocyclic couplers, and also the addition salts thereof.

Mention may be made, for example, of 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene, 2,6-bis(β-hydroxyethylamino)toluene, 6-hydroxyindoline, 2,6-dihydroxy-4-methylpyridine, 1-H-3-methylpyrazol-5-one, 1-phenyl-3-methylpyrazol-5-one, 2,6-dimethylpyrazolo[1,5-b]-1,2,4-triazole, 2,6-dimethyl[3,2-c]-1,2,4-triazole and 6-methylpyrazolo[1,5-a]benzimidazole, the addition salts thereof with an acid, and mixtures thereof.

The addition salts of the oxidation bases and couplers are especially chosen from the addition salts with an acid such as the hydrochlorides, hydrobromides, sulfates, citrates, succinates, tartrates, lactates, tosylates, benzenesulfonates, phosphates and acetates.

The oxidation base(s) are each generally present in an amount of from 0.0001% to 10% by weight relative to the total weight of the composition, and preferably from 0.005% to 5% by weight relative to the total weight of the composition.

The coupler(s) each generally represent from 0.0001% to 10% by weight relative to the total weight of the composition, and preferably from 0.005% to 5% by weight relative to the total weight of the composition.

The composition according to the invention may contain synthetic or natural, cationic or nonionic, direct dyes.

Examples of particularly suitable direct dyes that may be mentioned include nitrobenzene dyes; azo direct dyes; azomethine direct dyes; methine direct dyes; azacarbocyanin direct dyes, for instance tetraazacarbocyanins (tetraazapentamethines); quinone and in particular anthraquinone, naphthoquinone or benzoquinone direct dyes; azine direct dyes; xanthene direct dyes; triarylmethane direct dyes; indoamine direct dyes; indigoid direct dyes; phthalocyanine direct dyes, porphyrin direct dyes and natural direct dyes, alone or as mixtures. In particular, mention may be made of direct dyes from among: azo; methine; carbonyl; azine; nitro (hetero)aryl; tri(hetero)arylmethane; porphyrin; phthalocyanine and natural direct dyes, alone or as mixtures.

When they are present, the direct dye(s) more particularly represent(s) from 0.0001% to 10% by weight and preferably from 0.005% to 5% by weight of the total weight of the composition.

The composition according to the invention comprises one or more surfactants. The surfactant(s) may be cationic, amphoteric, nonionic and/or anionic surfactants. The surfactants of use in the composition of the invention are surfactants known per se in the field of dyeing keratin fibres.

The amphoteric or zwitterionic surfactant(s) that may be used in the present invention may especially be optionally quaternized secondary or tertiary aliphatic amine derivatives, in which the aliphatic group is a linear or branched chain containing from 8 to 22 carbon atoms, the said amine derivatives containing at least one anionic group, for instance a carboxylate, sulfonate, sulfate, phosphate or phosphonate group. In particular, mention may be made of (C₈-C₂₀)alkylbetaines, sulfobetaines, (C₈-C₂₀ alkyl)amido(C₃-C₈ alkyl)betaines or (C₈-C₂₀ alkyl)amido(C₆-C₈ alkyl)sulfobetaines. Among the optionally quaternized, secondary or tertiary aliphatic amine derivatives that can be used, as defined above, mention may also be made of the compounds having the respective structures (VIII) and (VIII') below:

Rₐ-CONHCH₂CH₂⁻ N⁺(R_{b})(R_{c})(CH₂COO⁻) (VIII)

in which:
Rₐ represents a C₁₀-C₃₀ alkyl or alkenyl group derived from an acid,
Rₐ-COOH, preferably present in hydrolysed coconut oil, represents a heptyl, nonyl or undecyl group,
R_{b} represents a β-hydroxyethyl group, and
R_{c} represents a carboxymethyl group;
and

Rₐ'-CONHCH₂CH₂-N(B)(B') (VIII')

in which:
B represents -CH₂CH₂OX',
B' represents -(CH₂)_{z}-Y', with z = 1 or 2,
X' represents the group -CH₂-COOH, CH₂-COOZ', -CH₂CH₂-COOH, -CH₂CH₂-COOZ', or a hydrogen atom,
Y' represents -COOH, -COOZ', the group -CH₂-CHOH-SO₃H or -CH₂-CHOH-SO₃Z',
Z' represents an ion derived from an alkali metal or alkaline-earth metal, such as sodium, an ammonium ion or an ion derived from an organic amine,
Rₐ' represents a C₁₀-C₃₀ alkyl or alkenyl group of an acid Rₐ'-COOH preferably present in coconut oil or in hydrolysed linseed oil, an alkyl group, especially a C₁₇ alkyl group, and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Among the amphoteric or zwitterionic surfactants mentioned above, use is preferably made of betaines comprising at least one saturated or unsaturated, C₈-C₃₀ fatty chain, and in particular the compounds of formula (A):

R₁-(CONH)ₓ-A₁- N⁺(R₂)(R₃)-A₂-Z (A)

with
x denoting 0 or 1,
A₁ and A₂ denoting, independently of one another, a linear or branched C₁-C₁₀ alkylene radical optionally substituted with a hydroxyl radical,
R₁ denoting a linear or branched C₆-C₃₀ alkyl or alkenyl radical,
R₂ and R₃ denoting, independently of one another, a linear or branched C₁-C₄ alkyl radical,
Z denoting a CO₂⁻ group or an SO₃⁻ group.

Preferably, R₂ and R₃ denote a methyl radical.

The amphoteric surfactant(s) of betaine type used in the cosmetic composition according to the present invention may especially be (C₈₋₂₀)alkylbetaines, (C₈₋₂₀)alkylsulfobetaines, (C₈₋₂₀ alkyl)amido(C₂₋₈ alkyl)betaines or (C₈₋₂₀ alkyl)amido(C₆₋₈ alkyl)sulfobetaines.

Among the amphoteric surfactants mentioned above that are preferably used are (C₈₋₂₀ alkyl)betaines and (C₈₋₂₀ alkyl)amido(C₂₋₈ alkyl)betaines, and mixtures thereof.

More particularly, the amphoteric surfactants of betaine type are selected from cocobetaine and cocamidopropylbetaine.

The term "anionic surfactant" means a surfactant comprising, as ionic or ionizable groups, only anionic groups. These anionic groups are preferably chosen from the following groups: CO₂H CO₂⁻, SO₃H SO₃⁻, OSO₃H, OSO₃⁻, H₂PO₃, HPO₃⁻, PO₃²⁻, H₂PO₂, HPO₂, HPO₂⁻, PO₂⁻, POH, PO⁻.

As examples of anionic surfactants that may be used in the composition according to the invention, mention may be made of alkyl sulfates, alkyl ether sulfates, alkylamido ether sulfates, alkylaryl polyether sulfates, monoglyceride sulfates, alkyl sulfonates, alkylamide sulfonates, alkylaryl sulfonates, alpha-olefin sulfonates, paraffin sulfonates, alkyl sulfosuccinates, alkyl ether sulfosuccinates, alkylamide sulfosuccinates, alkyl sulfoacetates, acyl sarcosinates, acyl glutamates, alkyl sulfosuccinamates, acyl isethionates and N-acyltaurates, salts of alkyl monoesters of polyglycoside-polycarboxylic acids, acyl lactylates, D-galactoside-uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds comprising from 6 to 24 carbon atoms and the aryl group denoting a phenyl group.

These compounds may be oxyethylenated and then preferably comprise from 1 to 50 ethylene oxide units.

The salts of C₆-C₂₄ alkyl monoesters of polyglycoside-polycarboxylic acids may be chosen from C₆-C₂₄ alkyl polyglycoside citrates, C₆-C₂₄ alkyl polyglycoside tartrates and C₆-C₂₄ alkyl polyglycoside sulfosuccinates.

When the anionic surfactant(s) are in salt form, they may be chosen from alkali metal salts such as the sodium or potassium salt and preferably the sodium salt, the ammonium salts, the amine salts and in particular the amino alcohol salts or the alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

Among the anionic surfactants, it is preferred, according to the invention, to use alkyl sulfate salts and alkyl ether sulfate salts and mixtures thereof.

The term "cationic surfactant" means a surfactant that is positively charged when it is contained in the composition according to the invention. This surfactant may bear one or more positive permanent charges or may contain one or more cationizable functions within the composition according to the invention.

The cationic surfactant(s) that may be used according to the present invention are preferably chosen from optionally polyoxyalkylenated primary, secondary or tertiary fatty amines, or the salts thereof, quaternary ammonium salts, and mixtures thereof.

The fatty amines generally comprise at least one C₈-C₃₀ hydrocarbon-based chain. Among the fatty amines that can be used according to the invention, examples that may be mentioned include stearylamidopropyldimethylamine and distearylamine.

Examples of quaternary ammonium salts that may especially be mentioned include:
- those corresponding to the general formula (IX) below: 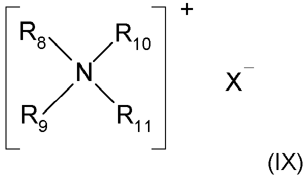 in which the groups R₈ to R₁₁, which may be identical or different, represent a linear or branched aliphatic group containing from 1 to 30 carbon atoms, or an aromatic group such as aryl or alkylaryl, at least one of the groups R₈ to R₁₁ denoting a group containing from 8 to 30 carbon atoms, preferably from 12 to 24 carbon atoms. The aliphatic groups may comprise heteroatoms such as, in particular, oxygen, nitrogen, sulfur and halogens. The aliphatic groups are chosen, for example, from C₁₋₃₀ alkyl, C₁₋₃₀ alkoxy, polyoxy(C₂-C₆)alkylene, C₁₋₃₀ alkylamide, (C₁₂-C₂₂)alkylamido(C₂-C₆)alkyl, (C₁₂-C₂₂)alkylacetate and C₁₋₃₀ hydroxyalkyl; X⁻ is an anion chosen from the group of halides, phosphates, acetates, lactates, (C₁-C₄)alkyl sulfates, and (C₁-C₄)alkyl- or (C₁-C₄)alkylaryl- sulfonates.
   Among the quaternary ammonium salts of formula (IX), those that are preferred are, on the one hand, tetraalkylammonium salts, for instance dialkyldimethylammonium or alkyltrimethylammonium salts in which the alkyl group contains approximately from 12 to 22 carbon atoms, in particular behenyltrimethylammonium, distearyldimethylammonium, cetyltrimethylammonium or benzyldimethylstearylammonium salts, or, on the other hand, the palmitylamidopropyltrimethylammonium salt, the stearamidopropyltrimethylammonium salt, the stearamidopropyldimethylcetearylammonium salt, or the stearamidopropyldimethyl(myristyl acetate)ammonium salt sold under the name Ceraphyl® 70 by the company Van Dyk. It is particularly preferred to use the chloride salts of these compounds;
- quaternary ammonium salts of imidazoline, for instance those of formula (X) below: 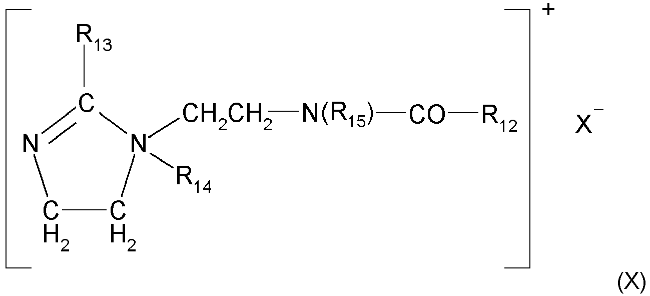 in which R₁₂ represents an alkyl or alkenyl group containing from 8 to 30 carbon atoms, derived for example from tallow fatty acids, R₁₃ represents a hydrogen atom, a C₁-C₄ alkyl group or an alkyl or alkenyl group containing from 8 to 30 carbon atoms, R₁₄ represents a C₁-C₄ alkyl group, R₁₅ represents a hydrogen atom or a C₁-C₄ alkyl group, X⁻ is an anion selected from the group consisting of halides, phosphates, acetates, lactates, alkyl sulfates, alkylsulfonates or alkylarylsulfonates in which the alkyl and aryl groups each preferably comprise from 1 to 20 carbon atoms and from 6 to 30 carbon atoms. R₁₂ and R₁₃ preferably denote a mixture of alkyl or alkenyl groups comprising from 12 to 21 carbon atoms, for example tallow fatty acid derivatives, R₁₄ denotes a methyl group, and R₁₅ denotes a hydrogen atom. Such a product is sold, for example, under the name Rewoquat® W 75 by the company Rewo;
- quaternary diammonium or triammonium salts, in particular of formula (XI): 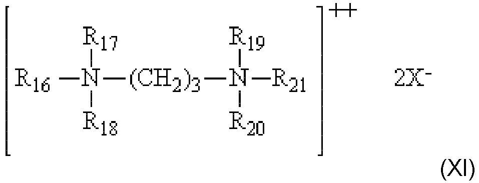 in which R₁₆ denotes an alkyl radical containing approximately from 16 to 30 carbon atoms, which is optionally hydroxylated and/or interrupted by one or more oxygen atoms, R₁₇ is selected from hydrogen and an alkyl radical containing from 1 to 4 carbon atoms or a group (R₁₆ₐ)(R₁₇ₐ)(R₁₈ₐ)N-(CH₂)₃, R₁₆ₐ, R₁₇ₐ, P₁₈ₐ, R₁₈, R₁₉, R₂₀ and R₂₁, which are identical or different, are selected from hydrogen and an alkyl radical containing from 1 to 4 carbon atoms, and X- is an anion selected from the group of halides, acetates, phosphates, nitrates and methyl sulfates. Such compounds are, for example, Finquat CT-P, available from the company Finetex (Quaternium 89), and Finquat CT, available from the company Finetex (Quaternium 75),
- quaternary ammonium salts containing at least one ester function, such as those of formula (XII) below: 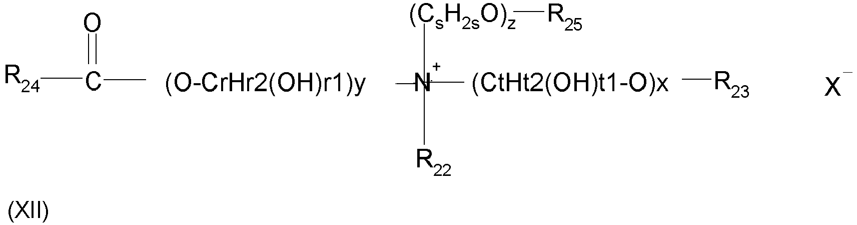 in which:
   R₂₂ is selected from C₁-C₆ alkyl groups and C₁-C₆ hydroxyalkyl or dihydroxyalkyl groups;
   R₂₃ is selected from:
      - the group 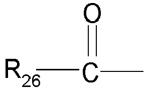
      - groups R₂₇, which are linear or branched, saturated or unsaturated C₁-C₂₂ hydrocarbon-based groups,
      - a hydrogen atom,
   R₂₅ is selected from:
      - the group 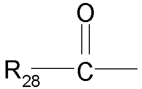
      - groups R₂₉, which are linear or branched, saturated or unsaturated C₁-C₆ hydrocarbon-based groups,
      - a hydrogen atom,
   R₂₄, R₂₆ and R₂₈, which are identical or different, are selected from linear or branched, saturated or unsaturated C₇-C₂₁ hydrocarbon radicals;
   r, s and t, which may be identical or different, are integers ranging from 2 to 6;
   r1 and t1, which may be identical or different, are equal to 0 or 1,
   and r2+r1=2r and t1+t2=2t,
   y is an integer ranging from 1 to 10;
   x and z, which may be identical or different, are integers ranging from 0 to 10;
   X- is a simple or complex, organic or inorganic anion;
   with the proviso that the sum x + y + z is from 1 to 15, that when x is 0, then R₂₃ denotes R₂₇ and that when z is 0, then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z is from 1 to 10.

When R₂₃ is a hydrocarbon-based group R₂₇, it may be long and may have 12 to 22 carbon atoms, or may be short and may have from 1 to 3 carbon atoms.

When R₂₅ is a hydrocarbon-based group R₂₉, it preferably contains 1 to 3 carbon atoms.

Advantageously, R₂₄, R₂₆ and R₂₈, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

Preferably, x and z, which may be identical or different, are equal to 0 or 1.

y is advantageously equal to 1.

Preferably, r, s and t, which may be identical or different, equal 2 or 3, and even more particularly are equal to 2.

The anion X- is preferably a halide (chloride, bromide or iodide) or an alkyl sulfate, more particularly methyl sulfate. However, it is possible to use methanesulfonate, phosphate, nitrate, tosylate, an anion derived from an organic acid, such as acetate or lactate, or any other anion that is compatible with the ammonium containing an ester function.

The anion X- is even more particularly chloride or methyl sulfate.

Use is made more particularly, in the composition according to the invention, of the ammonium salts of formula (XII) in which:
R²² denotes a methyl or ethyl group,
x and y are equal to 1;
z is equal to 0 or 1;
r, s and t are equal to 2;
R₂₃ is selected from:
   - the group 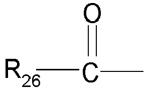
   - methyl, ethyl or C₁₄-C₂₂ hydrocarbon-based groups,
   - a hydrogen atom,
R₂₅ is selected from:
   - the group 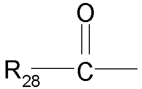
   - a hydrogen atom,
R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

The hydrocarbon-based groups are advantageously linear.

Mention may be made, for example, of the compounds of formula (XII) such as the diacyloxyethyldimethylammonium, diacyloxyethylhydroxyethylmethylammonium, monoacyloxyethyldihydroxyethylmethylammonium, triacyloxyethylmethylammonium and monoacyloxyethylhydroxyethyldimethylammonium salts (chloride or methyl sulfate in particular), and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, of triisopropanolamine, of an alkyldiethanolamine or of an alkyldiisopropanolamine, which are optionally oxyalkylenated, with C₁₀-C₃₀ fatty acids or with mixtures of C₁₀-C₃₀ fatty acids of plant or animal origin, or by transesterification of the methyl esters thereof. This esterification is followed by a quaternization using an alkylating agent such as an alkyl halide (preferably a methyl or ethyl halide), a dialkyl sulfate (preferably a dimethyl or diethyl sulfate), methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium monoester, diester and triester salts with a weight majority of diester salts.

It is also possible to use the ammonium salts containing at least one ester function that are described in patents US-A-4 874 554 and US-A-4 137 180.

Use may be made of behenoylhydroxypropyltrimethylammonium chloride sold by KAO under the name Quatarmin BTC 131.

Preferably, the ammonium salts containing at least one ester function contain two ester functions.

Among the quaternary ammonium salts containing at least one ester function, which can be used, it is preferred to use dipalmitoylethylhydroxyethylmethylammonium salts.

The non-ionic surfactants are more particularly chosen from mono-oxyalkylenated or polyoxyalkylenated and monoglycerolated or polyglycerolated non-ionic surfactants. The oxyalkylene units are more particularly oxyethylene or oxypropylene units, or a combination thereof, preferably oxyethylene units.

Examples of oxyalkylenated non-ionic surfactants that may be mentioned include:
- oxyalkylenated (C₈-C₂₄)alkylphenols,
- saturated or unsaturated, linear or branched, oxyalkylenated C₈-C₃₀ alcohols,
- saturated or unsaturated, linear or branched, oxyalkylenated C₈-C₃₀ amides,
- esters of saturated or unsaturated, linear or branched, C₈-C₃₀ acids and of polyethylene glycols,
- polyoxyethylenated esters of saturated or unsaturated, linear or branched, C₈-C₃₀ acids and of sorbitol,
- saturated or unsaturated, oxyethylenated plant oils,

The surfactants contain a number of moles of ethylene oxide and/or of propylene oxide of between 1 and 100, preferably between 2 and 50 and preferably between 2 and 30.

In accordance with one preferred embodiment of the invention, the oxyalkylenated nonionic surfactants are chosen from oxyethylenated C₈-C₃₀ alcohols comprising from 1 to 100 mol of ethylene oxide; polyoxyethylenated esters of linear or branched, saturated or unsaturated C₈-C₃₀ acids and of sorbitol comprising from 1 to 100 mol of ethylene oxide.

As examples of monoglycerolated or polyglycerolated non-ionic surfactants, monoglycerolated or polyglycerolated C₈-C₄₀ alcohols are preferably used.

In particular, the monoglycerolated or polyglycerolated C₈-C₄₀ alcohols correspond to the following formula:

RO-[CH₂-CH(CH₂OH)-O]ₘ-H

in which R represents a linear or branched C₈-C₄₀ and preferably C₈-C₃₀ alkyl or alkenyl radical, and m represents a number ranging from 1 to 30 and preferably from 1 to 10.

As examples of compounds that are suitable in the context of the invention, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleyl/cetyl alcohol containing 6 mol of glycerol, and octadecanol containing 6 mol of glycerol.

The alcohol may represent a mixture of alcohols in the same way that the value of m represents a statistical value, which means that, in a commercial product, several species of polyglycerolated fatty alcohols may coexist in the form of a mixture.

Among the monoglycerolated or polyglycerolated alcohols, it is more particularly preferred to use the C₈/Cₗₒ alcohol containing 1 mol of glycerol, the C₁₀/C₁₂ alcohol containing 1 mol of glycerol and the C₁₂ alcohol containing 1.5 mol of glycerol.

Nonionic surfactants that may also be mentioned include non-oxyethylenated fatty acid esters of sorbitan, fatty acid esters of sucrose, optionally oxyalkylenated alkylpolyglycosides, alkylglucoside esters, derivatives of N-alkylglucamine and of N-acylmethylglucamine, aldobionamides and amine oxides.

The composition of the invention preferably comprises one or more nonionic or anionic surfactants.

Even more preferentially, the composition of the invention comprises one or more nonionic surfactants.

The total content of surfactants in the composition of the invention is in general from 0.1% to 30% by weight, preferably from 1% to 20% by weight and better still from 2% to 10% by weight, relative to the weight of the composition.

The composition may also contain various adjuvants conventionally used in compositions for dyeing or lightening the hair, such as anionic polymers, cationic polymers other than those that are useful in the invention, and nonionic polymers, or mixtures thereof; antioxidants; penetrants; sequestrants; fragrances; dispersants; film-forming agents; ceramides; preserving agents; opacifiers.

The above adjuvants are generally present in an amount for each of them of between 0.01% and 20% by weight relative to the weight of the composition.

The composition according to the invention may comprise water and/or one or more organic solvents.

Examples of organic solvents that may be mentioned include linear or branched and preferably saturated monoalcohols or diols, comprising 2 to 10 carbon atoms, such as ethanol, isopropanol, hexylene glycol (2-methyl-2,4-pentanediol), neopentyl glycol and 3-methyl-1,5-pentanediol, butylene glycol, dipropylene glycol and propylene glycol; aromatic alcohols such as benzyl alcohol or phenylethyl alcohol; polyols containing more than two hydroxyl functions, such as glycerol; polyol ethers, for instance ethylene glycol monomethyl, monoethyl or monobutyl ether, propylene glycol or ethers thereof, for instance propylene glycol monomethyl ether; and also diethylene glycol alkyl ethers, especially C₁-C₄ alkyl ethers, for instance diethylene glycol monoethyl ether or monobutyl ether, alone or as a mixture.

The organic solvents, when they are present, generally represent between 1% and 40% by weight relative to the total weight of the dye composition, and preferably between 5% and 30% by weight relative to the total weight of the dye composition.

The composition is preferably aqueous. In this case, it preferably comprises from 30% to 95% by weight of water, better still from 40% to 90% by weight of water and even better still from 50% to 85% by weight of water relative to the total weight of the composition.

The pH of the composition according to the invention, if it is aqueous, is generally between 3 and 12, preferably between 5 and 11 and preferentially between 7 and 11, limits inclusive.

It may be adjusted to the desired value by means of acidifying or basifying agents usually used in the dyeing of keratin fibres, and in particular the aforementioned alkaline agents of the invention.

The composition, before dispersing in the form of a foam, may result from the mixing of two or more than two compositions.

The composition according to the invention is, on application to the keratin fibres, in the form of a foam.

The composition in foam form according to the invention is formed from a mixture of air or an inert gas with the composition described previously.

According to one particularly preferred embodiment, the composition according to the invention is in the form of a temporary foam produced just before use.

According to this embodiment, the composition may be packaged in a foam dispenser. They may either be products known as "aerosols" dispensed from a pressurized container with the aid of a propellent gas and thus forming a foam at the moment they are dispensed, or compositions dispensed from a container using a mechanical pump connected to a dispensing head, the passage of the composition into the dispensing head converting it into a foam at the latest at the outlet orifice of such a head.

The propellant gas that may be used may be chosen from carbon dioxide, nitrogen, nitrogen oxide, dimethyl ether, volatile hydrocarbons such as butane, isobutane, propane and pentane, and mixtures thereof.

The dispensing head is such that the substance that is sprayed in foam form is the composition according to the invention, i.e. the mixture of the composition with the oxidizing agent(s) and the composition with the oxidation dye precursor(s).

According to another embodiment, the composition may be in a foam dispenser of "pump-action bottle" type. These dispensers comprise a dispensing head for delivering the composition, a pump and a dip tube for transferring the composition from the container into the head in order to deliver the product. The foam is formed by forcing the composition to pass through a material comprising a porous substance such as a sintered material, a filtering grid made of plastic or of metal, or similar structures.

Such dispensers are well known to those skilled in the art and are described in patents: U.S. Pat No. 3 709 437 (Wright), U.S. Pat. No. 3 937 364 (Wright), U.S. Pat No. 4 022 351 (Wright), U.S. Pat No. 4 147 306 (Bennett), U.S. Pat No. 4 184 615 (Wright), U.S. Pat No. 4 598 862 (Rice), U.S. Pat No. 4 615 467 (Grogan et al.), and U.S. Pat No. 5 364 031 (Tamiguchi et al.).

In practice, for this variant, the oxidizing agent(s) are packaged in a first container equipped with a closure, and the oxidation dye precursor(s) are packaged in a second container, different from the first, and also closed by a closing member. The closing member may be a pump-dispensing mechanism. The composition according to the invention is then formed by mixing, before use, a composition with the oxidizing agent(s) and a composition with the oxidation dye precursor(s). To this end, to limit the number of containers provided, one from among the first and second container defines an internal volume that is sufficient to receive therein all of the two compositions. The mixture of the compositions may be homogenized by closing this container and by shaking the container. The closure of the container is advantageously carried out directly with the dispensing head. This dispensing head comprises a mechanical pump held in a ring intended for mounting by snap-fitting or screwing onto the neck of the container containing the mixture. The pump comprises a pump body connected to a dip tube to enable the whole of the mixture to be dispensed. The pump also comprises a push button for activation of the pump body, such that, on each activation, a dose of composition is sucked inside the dip tube and ejected in foam form out of the dispensing orifice of the head.

The containers are preferentially made of a thermoplastic material, and obtained via extrusion blow-moulding or injection blow-moulding processes. In particular, the container for conditioning the composition with the oxidation dye precursor(s) is made of a material comprising a non-zero proportion of EVOH. The pump is, for example, the standard "F2 - L9" model offered by the company Rexam.

According to this preferred embodiment, one subject of the invention is a non-aerosol device comprising the composition of the invention.

The dyeing process according to the invention consists in applying the composition according to the invention to wet or dry human keratin fibres over a time sufficient to develop the desired coloration. According to the invention, the composition applied to the keratin fibres is in foam form. The dyeing process is generally performed at room temperature (between 15 and 25°C) and up to temperatures that may be as high as 60°C to 80°C.

After a leave-on time of from one minute to one hour and preferably from 5 minutes to 30 minutes, the human keratin fibres are rinsed with water, and optionally washed with a shampoo and then rinsed with water.

The examples that follow serve to illustrate the invention without, however, being limiting in nature.

### EXAMPLES

The following compositions are prepared (the amounts are expressed in g% of active material):

| Ingredients | Amount (g) |
|---|---|
| Ammonium thiolactate | 0.2 |
| Aqueous ammonia (expressed as NH₃) | 0.8 |
| Erythorbic acid | 0.1 |
| Monoethanolamine | 2 |
| Ethylenediaminetetraacetic acid | 0.1 |
| Oleyl alcohol | 0.5 |
| Fragrance | 0.4 |
| Polydimethyldiallylammonium chloride (Polyquaternium-6) | 0.5 |
| Hexylene glycol (2-methyl-2,4-pentanediol) | 2 |
| Dipropylene glycol | 2 |
| 96° ethyl alcohol | 3 |
| Propylene glycol | 2 |
| Oxyethylenated decyl alcohol (3 OE) | 4 |
| Protected oxyethylenated (4 OE) rapeseed acid amide (INCI: PEG-4 rapeseed amide) | 3 |
| Lauryl ether carboxylic acid (4.5 OE) | 2 |
| Glyceryl C12 alkyl ether (1.5 mol) (INCI: glyceryl lauryl ether) | 3 |
| Condensate of ethylene oxide and of propylene oxide and ethylene oxide (MW: 14000) (128 OE/54 OP/128 OE) (INCI: Poloxamer 338) | 1 |
| Hydrogen peroxide | 4.5 |
| Phosphoric acid | qs |
| Etidronic acid, tetrasodium salt | 0.04 |
| Tetrasodium pyrophosphate | 0.02 |
| Sodium salicylate | 0.02 |
| Glycerol | 2 |
| Laureth-11 | 1 |
| 1,4-Diaminobenzene | 0.8 |
| 1-β-Hydroxyethyloxy-2,4-diaminobenzene dihydrochloride | 0.04 |
| 1N,N-Bis(2-hydroxyethyl)amino-4-aminobenzene sulfate monohydrate | 0.18 |
| 2-Methyl-1,3-dihydroxybenzene | 0.028 |
| 1,3-Dihydroxybenzene | 0.68 |
| 1-Hydroxy-3-aminobenzene | 0.24 |
| Water | qs 100 |

The above dye composition is obtained by mixing, before use, the following two compositions in a composition A/composition B weight ratio of 0.666.

| **Composition A** | **% by weight** |
|---|---|
| Ammonium thiolactate | 0.5 |
| Aqueous ammonia (expressed as NH₃) | 2 |
| Erythorbic acid | 0.25 |
| Monoethanolamine | 5 |
| Ethylenediaminetetraacetic acid | 0.25 |
| Oleyl alcohol | 1.25 |
| Fragrance | 1 |
| Polydimethyldiallylammonium chloride (Polyquaternium-6) | 1.25 |
| Hexylene glycol (2-methyl-2,4-pentanediol) | 5 |
| Dipropylene glycol | 5 |
| 96° ethyl alcohol | 7.5 |
| Propylene glycol | 5 |
| Oxyethylenated decyl alcohol (3 OE) | 10 |
| Protected oxyethylenated (4 OE) rapeseed acid amide (INCI: PEG-4 rapeseed amide) | 7.5 |
| Lauryl ether carboxylic acid (4.5 OE) | 5 |
| Glyceryl C12 alkyl ether (1.5 mol) (INCI: glyceryl lauryl ether) | 7.5 |
| Condensate of ethylene oxide and of propylene oxide and ethylene oxide (MW: 14000) (128 OE/54 OP/128 OE) (INCI: Poloxamer 338) | 2.5 |
| 1,4-Diaminobenzene | 2 |
| 1-β-Hydroxyethyloxy-2,4-diaminobenzene dihydrochloride | 0.1 |
| 1N,N-Bis(2-hydroxyethyl)amino-4-aminobenzene sulfate monohydrate | 0.45 |
| 2-Methyl-1,3-dihydroxybenzene | 0.07 |
| 1,3-Dihydroxybenzene | 1.7 |
| 1-Hydroxy-3-aminobenzene | 0.6 |
| Water | qs 100 |

| **Composition B** | **% by weight** |
|---|---|
| Glycerol | 4 |
| Etidronic acid, tetrasodium salt | 0.06 |
| Tetrasodium pyrophosphate | 0.04 |
| Sodium salicylate | 0.035 |
| Hydrogen peroxide | 7.5 |
| Laureth-11 | 1 |
| Phosphoric acid | qs pH 2.2 |
| Water | qs 100 |

The mixture is introduced in an amount of 65 g (26 g of composition A + 39 g of composition B) into a pump bottle (Rexam L9 equipped with a dip tube). The device produces a compact foam on pumping. This compact foam is applied to natural or permanent-waved grey hair containing 90% white hairs, without disintegrating immediately on application. The comfort on application is very good. After a leave-in time of 30 minutes, the locks are rinsed, washed with a standard shampoo, rinsed again and then dried, to give a dark chestnut-brown coloration. This coloration is strong and sparingly selective.

The propellant gas that may be used may be chosen from carbon dioxide, nitrogen, nitrogen oxide, dimethyl ether, volatile hydrocarbons such as butane, isobutane, propane and pentane, and mixtures thereof.

The dispensing head is such that the substance that is sprayed in foam form is the composition according to the invention, i.e. the mixture of the composition with the oxidizing agent(s) and the composition with the oxidation dye precursor(s).

According to another embodiment, the composition may be in a foam dispenser of "pump-action bottle" type. These dispensers comprise a dispensing head for delivering the composition, a pump and a dip tube for transferring the composition from the container into the head in order to deliver the product. The foam is formed by forcing the composition to pass through a material comprising a porous substance such as a sintered material, a filtering grid made of plastic or of metal, or similar structures.

Such dispensers are well known to those skilled in the art and are described in patents: U.S. Pat No. 3 709 437 (Wright), U.S. Pat. No. 3 937 364 (Wright), U.S. Pat No. 4 022 351 (Wright), U.S. Pat No. 4 147 306 (Bennett), U.S. Pat No. 4 184 615 (Wright), U.S. Pat No. 4 598 862 (Rice), U.S. Pat No. 4 615 467 (Grogan et al.), and U.S. Pat No. 5 364 031 (Tamiguchi et al.).

In practice, for this variant, the oxidizing agent(s) are packaged in a first container equipped with a closure, and the oxidation dye precursor(s) are packaged in a second container, different from the first, and also closed by a closing member. The closing member may be a pump-dispensing mechanism. The composition according to the invention is then formed by mixing, before use, a composition with the oxidizing agent(s) and a composition with the oxidation dye precursor(s). To this end, to limit the number of containers provided, one from among the first and second container defines an internal volume that is sufficient to receive therein all of the two compositions. The mixture of the compositions may be homogenized by closing this container and by shaking the container. The closure of the container is advantageously carried out directly with the dispensing head. This dispensing head comprises a mechanical pump held in a ring intended for mounting by snap-fitting or screwing onto the neck of the container containing the mixture. The pump comprises a pump body connected to a dip tube to enable the whole of the mixture to be dispensed. The pump also comprises a push button for activation of the pump body, such that, on each activation, a dose of composition is sucked inside the dip tube and ejected in foam form out of the dispensing orifice of the head.

The containers are preferentially made of a thermoplastic material, and obtained via extrusion blow-moulding or injection blow-moulding processes. In particular, the container for conditioning the composition with the oxidation dye precursor(s) is made of a material comprising a non-zero proportion of EVOH. The pump is, for example, the standard "F2 - L9" model offered by the company Rexam.

According to this preferred embodiment, one subject of the invention is a non-aerosol device comprising the composition of the invention.

The dyeing process according to the invention consists in applying the composition according to the invention to wet or dry human keratin fibres over a time sufficient to develop the desired coloration. According to the invention, the composition applied to the keratin fibres is in foam form. The dyeing process is generally performed at room temperature (between 15 and 25°C) and up to temperatures that may be as high as 60°C to 80°C.

After a leave-on time of from one minute to one hour and preferably from 5 minutes to 30 minutes, the human keratin fibres are rinsed with water, and optionally washed with a shampoo and then rinsed with water.

The examples that follow serve to illustrate the invention without, however, being limiting in nature.

### EXAMPLES

The following compositions are prepared (the amounts are expressed in g% of active material):

| Ingredients | Amount (g) |
|---|---|
| Ammonium thiolactate | 0.2 |
| Aqueous ammonia (expressed as NH₃) | 0.8 |
| Erythorbic acid | 0.1 |
| Monoethanolamine | 2 |
| Ethylenediaminetetraacetic acid | 0.1 |
| Oleyl alcohol | 0.5 |
| Fragrance | 0.4 |
| Polydimethyldiallylammonium chloride (Polyquaternium-6) | 0.5 |
| Hexylene glycol (2-methyl-2,4-pentanediol) | 2 |
| Dipropylene glycol | 2 |
| 96° ethyl alcohol | 3 |
| Propylene glycol | 2 |
| Oxyethylenated decyl alcohol (3 OE) | 4 |
| Protected oxyethylenated (4 OE) rapeseed acid amide (INCI: PEG-4 rapeseed amide) | 3 |
| Lauryl ether carboxylic acid (4.5 OE) | 2 |
| Glyceryl C12 alkyl ether (1.5 mol) (INCI: glyceryl lauryl ether) | 3 |
| Condensate of ethylene oxide and of propylene oxide and ethylene oxide (MW: 14000) (128 OE/54 OP/128 OE) (INCI: Poloxamer 338) | 1 |
| Hydrogen peroxide | 4.5 |
| Phosphoric acid | qs |
| Etidronic acid, tetrasodium salt | 0.04 |
| Tetrasodium pyrophosphate | 0.02 |
| Sodium salicylate | 0.02 |
| Glycerol | 2 |
| Laureth-11 | 1 |
| 1,4-Diaminobenzene | 0.8 |
| 1-β-Hydroxyethyloxy-2,4-diaminobenzene dihydrochloride | 0.04 |
| 1N,N-Bis(2-hydroxyethyl)amino-4-aminobenzene sulfate monohydrate | 0.18 |
| 2-Methyl-1,3-dihydroxybenzene | 0.028 |
| 1,3-Dihydroxybenzene | 0.68 |
| 1-Hydroxy-3-aminobenzene | 0.24 |
| Water | qs 100 |

The above dye composition is obtained by mixing, before use, the following two compositions in a composition A/composition B weight ratio of 0.666.

| **Composition A** | **% by weight** |
|---|---|
| Ammonium thiolactate | 0.5 |
| Aqueous ammonia (expressed as NH₃) | 2 |
| Erythorbic acid | 0.25 |
| Monoethanolamine | 5 |
| Ethylenediaminetetraacetic acid | 0.25 |
| Oleyl alcohol | 1.25 |
| Fragrance | 1 |
| Polydimethyldiallylammonium chloride (Polyquaternium-6) | 1.25 |
| Hexylene glycol (2-methyl-2,4-pentanediol) | 5 |
| Dipropylene glycol | 5 |
| 96° ethyl alcohol | 7.5 |
| Propylene glycol | 5 |
| Oxyethylenated decyl alcohol (3 OE) | 10 |
| Protected oxyethylenated (4 OE) rapeseed acid amide (INCI: PEG-4 rapeseed amide) | 7.5 |
| Lauryl ether carboxylic acid (4.5 OE) | 5 |
| Glyceryl C12 alkyl ether (1.5 mol) (INCI: glyceryl lauryl ether) | 7.5 |
| Condensate of ethylene oxide and of propylene oxide and ethylene oxide (MW: 14000) (128 OE/54 OP/128 OE) (INCI: Poloxamer 338) | 2.5 |
| 1,4-Diaminobenzene | 2 |
| 1-β-Hydroxyethyloxy-2,4-diaminobenzene dihydrochloride | 0.1 |
| 1N,N-Bis(2-hydroxyethyl)amino-4-aminobenzene sulfate monohydrate | 0.45 |
| 2-Methyl-1,3-dihydroxybenzene | 0.07 |
| 1,3-Dihydroxybenzene | 1.7 |
| 1-Hydroxy-3-aminobenzene | 0.6 |
| Water | qs 100 |

| **Composition B** | **% by weight** |
|---|---|
| Glycerol | 4 |
| Etidronic acid, tetrasodium salt | 0.06 |
| Tetrasodium pyrophosphate | 0.04 |
| Sodium salicylate | 0.035 |
| Hydrogen peroxide | 7.5 |
| Laureth-11 | 1 |
| Phosphoric acid | qs pH 2.2 |
| Water | qs 100 |

The mixture is introduced in an amount of 65 g (26 g of composition A + 39 g of composition B) into a pump bottle (Rexam L9 equipped with a dip tube). The device produces a compact foam on pumping. This compact foam is applied to natural or permanent-waved grey hair containing 90% white hairs, without disintegrating immediately on application. The comfort on application is very good. After a leave-in time of 30 minutes, the locks are rinsed, washed with a standard shampoo, rinsed again and then dried, to give a dark chestnut-brown coloration. This coloration is strong and sparingly selective.

## Claims

1. Dye composition in foam form comprising at least one oxidation dye precursor, at least one oxidizing agent, at least one alkaline agent selected from ammonia and monoethanolamine, and mixtures thereof, at least one liquid fatty alcohol containing from 8 to 30 carbon atoms and at least one cationic polymer with a cationic charge density of greater than 4 meq./g corresponding to homopolymers or copolymers containing, as main constituent of the chain, units corresponding to formula (I) or (II): 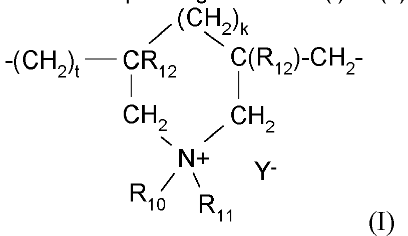 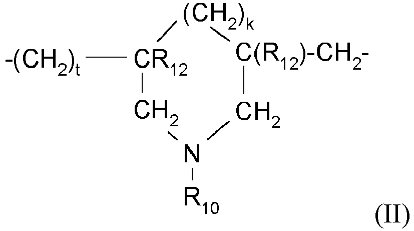 in which formulae k and t are equal to 0 or 1, the sum k + t being equal to 1; R12 denotes a hydrogen atom or a methyl radical; R10 and R11, independently of each other, denote an alkyl group containing from 1 to 6 carbon atoms.

2. Composition according to the preceding claim, **characterized in that** it comprises as oxidation dye precursors one or more oxidation bases chosen from para-phenylenediamines, bis(phenyl)alkylenediamines, para-aminophenols, ortho-aminophenols and heterocyclic bases, and the addition salts thereof, and optionally one or more couplers chosen from meta-phenylenediamines, meta-aminophenols, meta-diphenols, naphthalene-based couplers and heterocyclic couplers, and also the addition salts thereof.

3. Composition according to either of the preceding claims, in which the oxidizing agent is hydrogen peroxide.

4. Composition according to any one of the preceding claims, in which the liquid fatty alcohol is chosen from saturated fatty alcohols and in particular from octyldodecanol, isostearyl alcohol and 2-hexyldecanol.

5. Composition according to any one of Claims 1 to 4, in which the liquid fatty alcohol is chosen from unsaturated fatty alcohols and in particular from oleyl alcohol, linoleyl alcohol, linolenyl alcohol and undecylenyl alcohol, preferably oleyl alcohol.

6. Composition according to any one of the preceding claims, in which the liquid fatty alcohol(s) are present in an amount ranging from 0.1 % to 20%, preferably from 0.2% to 10% and better still from 0.3% to 5% by weight, relative to the total weight of the composition.

7. Composition according to any one of the preceding claims, in which the amount of cationic polymer(s) with a cationic charge density of greater than 4 meq./g ranges from 0.01% to 10%, better still from 0.1% to 5% and even better still from 0.2% to 2% by weight relative to the total weight of the composition.

8. Composition according to any one of the preceding claims, comprising at least one anionic or nonionic surfactant, preferably a nonionic surfactant.

9. Hair dyeing process, **characterized in that** a composition comprising at least one dye precursor, at least one alkaline agent, at least one liquid fatty alcohol and at least one cationic polymer with a cationic charge density of greater than 4 meq./g is mixed with an oxidizing agent, and the mixture is then applied in foam form onto the hair; the dye precursor, the alkaline agent, liquid fatty alcohol, the cationic polymer with a cationic charge density of greater than 4 meq./g and the oxidizing agent being as defined in any one of Claims 1 to 8.

10. Aerosol device comprising a means for generating in foam form a composition as defined in any one of Claims 1 to 8.

11. Non-aerosol device comprising a bottle equipped with a mechanical pumping system and comprising a composition as defined in any one of Claims 1 to 8 in liquid form and a dispensing system for delivering it in foam form.

12. Two-compartment or three-compartment device comprising, in a first compartment, a first composition containing at least one oxidation dye precursor, at least one alkaline agent, at least one liquid fatty alcohol and at least one cationic polymer with a cationic charge density of greater than 4 meq./g; in a second compartment, a second composition containing one or more oxidizing agents, one of the two compartments being equipped with a component for delivering the composition of the invention in foam form after mixing with the other composition, or the component for delivering the composition in foam form is included in a third compartment; the dye precursor, the alkaline agent, liquid fatty alcohol and the cationic polymer with a cationic charge density of greater than 4 meq./g and the oxidizing agent being as defined in any one of Claims 1 to 8.

## Patentansprüche

1. Farbstoffzusammensetzung in Schaumform, umfassend mindestens eine Oxidationsfarbstoffvorstufe, mindestens ein Oxidationsmittel, mindestens ein alkalisches Mittel, das aus Ammoniak und Monoethanolamin und Mischungen davon ausgewählt ist, mindestens einen flüssigen Fettalkohol mit 8-30 Kohlenstoffatomen und mindestens ein kationisches Polymer mit einer kationischen Ladungsdichte von mehr als 4 meq/g, das Homopolymeren oder Copolymeren entspricht, die als Hauptbestandteil der Kette Einheiten der Formel (I) oder (II) enthalten: 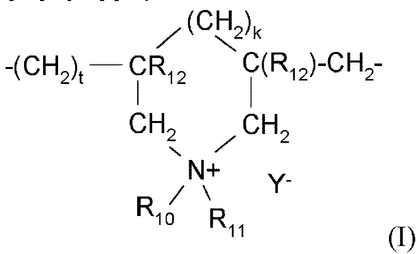 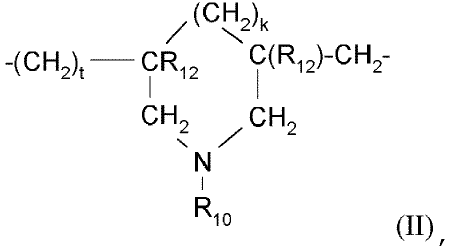 wobei in den Formeln k und t gleich 0 oder 1 sind, die Summe k + t gleich 1 ist; R12 für ein Wasserstoffatom oder einen Methylrest steht; R10 und R11 unabhängig voneinander für eine Alkylgruppe mit 1 bis 6 Kohlenstoffatomen stehen.

2. Zusammensetzung nach dem vorhergehenden Anspruch, **dadurch gekennzeichnet, dass** sie als Oxidationsfarbstoffvorstufen eine oder mehrere Oxidationsbasen, die aus para-Phenylendiaminen, Bis(phenyl)-alkylendiaminen, para-Aminophenolen, ortho-Aminophenolen und heterocyclischen Basen und den Additionssalzen davon ausgewählt sind, und gegebenenfalls einen oder mehrere Kuppler, die aus meta-Phenylendiaminen, meta-Aminophenolen, meta-Diphenolen, auf Naphthalin basierenden Kupplern, heterocyclischen Kupplern sowie den Additionssalzen davon ausgewählt sind, umfasst.

3. Zusammensetzung nach einem der vorhergehenden Ansprüche, wobei es sich bei dem Oxidationsmittel um Wasserstoffperoxid handelt.

4. Zusammensetzung nach einem der vorhergehenden Ansprüche, wobei der flüssige Fettalkohol aus gesättigten Fettalkoholen und insbesondere aus Octyldodecanol, Isostearylalkohol und 2-Hexyldecanol ausgewählt ist.

5. Zusammensetzung nach einem der Ansprüche 1 bis 4, wobei der flüssige Fettalkohol aus ungesättigten Fettalkoholen und insbesondere aus Oleylalkohol, Linoleylalkohol, Linolenylalkohol und Undecylenylalkohol, vorzugsweise Oleylalkohol, ausgewählt ist.

6. Zusammensetzung nach einem der vorhergehenden Ansprüche, wobei der flüssige Fettalkohol bzw. die flüssigen Fettalkohole in einer Menge im Bereich von 0,1 bis 20 Gew.-%, vorzugsweise von 0,2 bis 10 Gew.-% und noch besser von 0,3 bis 5 Gew.-%, bezogen auf das Gesamtgewicht der Zusammensetzung, vorliegt bzw. vorliegen.

7. Zusammensetzung nach einem der vorhergehenden Ansprüche, wobei die Menge an kationischem Polymer bzw. kationischen Polymeren mit einer kationischen Ladungsdichte von mehr als 4 meq/g im Bereich von 0,01 bis 10 Gew.-%, noch besser von 0,1 bis 5 Gew.-% und sogar noch besser von 0,2 bis 2 Gew.-%, bezogen auf das Gesamtgewicht der Zusammensetzung, liegt.

8. Zusammensetzung nach einem der vorhergehenden Ansprüche, umfassend mindestens ein anionisches oder nichtionisches Tensid, vorzugsweise ein nichtionisches Tensid.

9. Haarfärbeverfahren, **dadurch gekennzeichnet, dass** man eine Zusammensetzung, die mindestens eine Farbstoffvorstufe, mindestens ein alkalisches Mittel, mindestens einen flüssigen Fettalkohol und mindestens ein kationisches Polymer mit einer kationischen Ladungsdichte von mehr als 4 meq/g umfasst, mit einem Oxidationsmittel mischt und die Mischung dann in Schaumform auf das Haar aufbringt; wobei die Farbstoffvorstufe, das alkalische Mittel, der flüssige Fettalkohol, das kationische Polymer mit einer kationischen Ladungsdichte von mehr als 4 meq/g und das Oxidationsmittel wie in einem der Ansprüche 1 bis 8 definiert sind.

10. Aerosolvorrichtung mit einem Mittel zur Erzeugung einer Zusammensetzung gemäß einem der Ansprüche 1 bis 8 in Schaumform.

11. Nichtaerosolvorrichtung mit einer Flasche, die mit einem mechanischen Pumpsystem ausgestattet ist und eine Zusammensetzung gemäß einem der Ansprüche 1 bis 8 in flüssiger Form umfasst, und einem Spendersystem zur Abgabe der Zusammensetzung in Schaumform.

12. Vorrichtung mit 2 oder 3 Kompartimenten, umfassend in einem ersten Kompartiment eine erste Zusammensetzung, die mindestens eine Oxidationsfarbstoffvorstufe, mindestens ein alkalisches Mittel, mindestens einen flüssigen Fettalkohol und mindestens ein kationisches Polymer mit einer kationischen Ladungsdichte von mehr als 4 meq/g enthält; in einem zweiten Kompartiment eine zweite Zusammensetzung, die ein oder mehrere Oxidationsmittel enthält, wobei eines der beiden Kompartimente mit einer Komponente zur Abgabe der erfindungsgemäßen Zusammensetzung in Schaumform nach Mischen mit der anderen Zusammensetzung ausgestattet ist oder die Komponente zur Abgabe der Zusammensetzung in Schaumform in einem dritten Kompartiment enthalten ist; wobei die OxidationsFarbstoffvorstufe, das alkalische Mittel, der flüssige Fettalkohol, das kationische Polymer mit einer kationischen Ladungsdichte von mehr als 4 meq/g und das Oxidationsmittel wie in einem der Ansprüche 1 bis 8 definiert sind.

## Revendications

1. Composition de coloration sous forme de mousse comprenant au moins un précurseur de colorant d'oxydation, au moins un agent oxydant, au moins un agent alcalin choisi parmi l'ammoniaque, la monoéthanolamine et leurs mélanges, au moins un alcool gras liquide contenant de 8 à 30 atomes de carbone et au moins un polymère cationique de densité de charges cationiques supérieure à 4 meq/g tels que les homopolymères ou copolymères comportant comme constituant principal de la chaîne des motifs répondant aux formules (I) ou (II) : 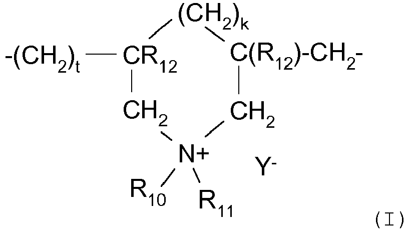 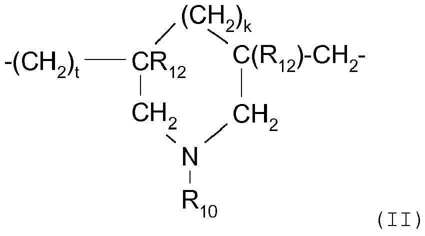 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone.

2. Composition selon la revendication précédente, **caractérisée en ce qu'**elle comprend à titre de précurseurs de colorant d'oxydation, une ou plusieurs bases d'oxydation choisies parmi les paraphénylènediamines, les bis-phénylalkylènediamines, les para-aminophénols, les ortho-aminophénols, les bases hétérocycliques et leurs sels d'addition et éventuellement un ou plusieurs coupleurs choisis parmi les méta-phénylènediamines, les méta-aminophénols, les méta-diphénols, les coupleurs naphtaléniques, les coupleurs hétérocycliques ainsi que leurs sels d'addition.

3. Composition selon l'une quelconque des revendications précédentes dans laquelle l'agent oxydant est le peroxyde d'hydrogène.

4. Composition selon l'une quelconque des revendications précédentes dans laquelle l'alcool gras liquide est choisi parmi les alcools gras saturés et en particulier parmi parmi l'octyldodécanol, l'alcool isostéarylique, le 2-hexyldécanol.

5. Composition selon l'une quelconque des revendications 1 à 6 dans laquelle l'alcool gras liquide est choisi parmi les alcools gras insaturés et en particulier parmi l'alcool oléique (ou oléylique), l'alcool linoléique (ou linoléylique), l'alcool linolénique (ou linolénylique), l'alcool undécylénique, de préférence l'alcool oléique(ou oléylique).

6. Composition selon l'une quelconque des revendications précédentes dans laquelle le ou les alcools gras liquides sont présents en une quantité allant de 0,1 à 20 % , de préférence de 0,2 à 10%, encore mieux de 0,3 à 5% en poids par rapport au poids total de la composition

7. Composition selon l'une quelconque des revendications précédentes dans laquelle la quantité de polymère(s) cationique(s) à densité de charges cationiques supérieure à 4 meq/g varie de 0,01 à 10%, mieux de 0,1 à 5%, encore mieux de 0,2 à 2% en poids par rapport au poids total de la composition.

8. Composition selon l'une quelconque des revendications précédentes comprenant au moins un tensioactif anionique ou non ionique, de préférence non ionique.

9. Procédé de coloration des cheveux, **caractérisé en ce que** l'on mélange une composition comprenant au moins un précurseur de colorant, au moins un agent alcalin, au moins un alcool gras liquide et au moins un polymère cationique à densité de charges cationiques supérieure à 4 meq/g avec un agent oxydant puis on applique le mélange sous forme de mousse sur les cheveux ; le précurseur de colorant, l'agent alcalin, l'alcool gras liquide et le polymère cationique à densité de charges cationiques supérieure à 4 meq/g et l'agent oxydant étant tels que définis à l'une quelconque des revendications 1 à 8.

10. Dispositif aérosol comprenant un moyen de générer sous forme de mousse une composition telle que définie à l'une quelconque des revendications 1 à 8.

11. Dispositif non aérosol comprenant un flacon équipé d'un système mécanique de pompage et comprenant une composition telle que définie à l'une quelconque des revendications 1 à 8 sous forme liquide et un système de distribution permettant de la délivrer sous forme de mousse.

12. Dispositif à deux ou trois compartiments comprenant, dans un premier compartiment, une première composition renfermant au moins un précurseur de colorant d'oxydation, au moins un agent alcalin, au moins un alcool gras liquide et au moins un polymère cationique à densité de charges cationiques supérieure à 4 meq/g ; dans un second compartiment, une seconde composition renfermant un ou plusieurs agents oxydants, l'un des deux compartiments étant muni d'un élément permettant la délivrance de la composition de l'invention sous forme d'une mousse après mélange avec l'autre composition ou l'élément permettant la délivrance de la composition sous forme de mousse est compris dans un troisième compartiment; le précurseur de colorant, l'agent alcalin, l'alcool gras liquide, le polymère cationique à densité de charges cationiques supérieure à 4 meq/g et l'agent oxydant étant tels que définis à l'une quelconque des revendications 1 à 8.
